# EUROPEAN PATENT APPLICATION

(11) **EP 2 990 110 A1**
(43) Date of publication of application: **02.03.2016**
(21) Application number: 14788730.1
(22) Date of filing: 17.04.2014
(51) Int. Cl.: B01J 23/92, B01J 23/30, B01J 38/00, C07D 301/12, C07D 303/04, C07D 303/44

(54) **RECOVERY METHOD AND REUSE METHOD OF OXO ACID CATALYST**

(30) Priority: 23.04.2013 JP 2013090355
(71) Applicant: Daicel Corporation, Osaka-shi, Osaka 530-0011 (JP)
(72) Inventor: KITAYAMA, Kenji, Himeji-shi Hyogo 671-1283 (JP); SHIBATA, Hikaru, Himeji-shi Hyogo 671-1283 (JP); SUZUKI, Takamasa, Himeji-shi Hyogo 671-1283 (JP); UEHARA, Kazuhiro, Himeji-shi Hyogo 671-1283 (JP)
(74) Representative: Grünecker Patent- und Rechtsanwälte PartG mbB
(86) International application number: PCT/JP2014/060899
(87) International publication number: WO 2014/175152

(57) **Abstract**

Provided is a method for easily and efficiently recovering an oxoacid catalyst without deterioration in reaction product yield and catalytic activity, where the oxoacid catalyst has been used in a reaction for oxidizing an organic compound with hydrogen peroxide. Also provided is a method for producing an oxide in which an organic compound is oxidized with hydrogen peroxide using the oxoacid catalyst recovered by the method, to yield the corresponding oxide.

A method according to the present invention recovers an oxoacid catalyst used in a reaction for oxidizing an organic compound with hydrogen peroxide in an aqueous/organic solvent two-phase system. The method includes Step 1 in which the pH in the reaction system is adjusted to 5.0 or higher so as to transfer the oxoacid catalyst to the aqueous phase, and the organic phase is removed.

## Description

### Technical Field

The present invention relates to methods for easily and efficiently separating/recovering an oxoacid catalyst used in a reaction for oxidizing an organic compound with hydrogen peroxide, and reusing the recovered oxoacid catalyst in an oxidation reaction of an organic compound. The present application claims priority to Japanese Patent Application No. 2013-090355 filed to Japan on April 23, 2013, the entire contents of which are incorporated herein by reference.

### Background Art

Oxidizing agents are used in oxidation reactions such as oxidation of primary alcohols to yield aldehydes and carboxylic acids, oxidation of secondary alcohols to yield ketones, and oxidation of unsaturated compounds to yield epoxy compounds and diols.

Of the oxidizing agents, hydrogen peroxide is inexpensive, is not corrosive, yields water as a by-product, can lighten the environmental load, and thereby receives attention. Disadvantageously, however, hydrogen peroxide, when used as an oxidizing agent, offers a low conversion from the reactant (reaction substrate), and a low selectivity of the reaction product. Hydrogen peroxide is therefore generally used in combination with a metal catalyst. Because of expensiveness of the metal catalyst, methods for recovering the metal catalyst after the completion of the reaction and reusing the reovered catalyst in another reaction have been investigated.

Patent Literature (PTL) 1 describes a method for adsorbing/separating a metal catalyst using a chelate resin. Disadvantageously, however, the method is insufficient in recovery rate. Further disadvantageously, the method requires a large amount of the chelate resin, invites high cost, and suffers from a low yield of the reaction product because the chelate resin also adsorbs the reaction product.

PTL 2, PTL3, and PTL 4 describe methods for using a metal catalyst as immobilized typically on a carrier in a reaction, and, after the completion of the reaction, separating/recovering the metal catalyst by filtration. Unfortunately, however, the methods are insufficient in recovery rate. Further unfortunately, such metal catalyst, when immobilized typically on a carrier, becomes insoluble in the reaction liquid and has a lower activity.

### Citation List

### Patent Literature

PTL 1: Japanese Unexamined Patent Application Publication (JP-A) No. H11-130762
PTL 2: JP-A No. 2001-17863
PTL 3: JP-A No. 2001-17864
PTL 4: JP-A No. 2002-59007

### Summary of Invention

### Technical Problem

Accordingly, the present invention has an object to provide a method for easily and efficiently recovering an oxoacid catalyst without adversely affecting the yield of a reaction product and the activity of the catalyst, where the oxoacid catalyst has been used in a reaction for oxidizing an organic compound with hydrogen peroxide.

The present invention has another object to provide a method for producing an oxide by oxidizing an organic compound with hydrogen peroxide using the oxoacid catalyst recovered by the method, and thereby yielding the corresponding oxide.

### Solution to Problem

After intensive investigations to achieve the objects, the present inventors have found followings. An oxoacid catalyst in an aqueous/organic solvent two-phase reaction system can be transferred from the organic phase to the aqueous phase, or from the aqueous phase to the organic phase by adjusting the pH in the reaction system. Thus, the oxoacid catalyst can be easily separated from a reaction product contained in the organic phase and can be recovered. In addition, impurities in the reaction system can be removed from the oxoacid catalyst by optionally transferring the oxoacid catalyst between the aqueous phase and the organic phase. Thus, the oxoacid catalyst can be purified and recovered. Such impurities include those having solubility in the organic solvent; and those having solubility in water. The recovered oxoacid catalyst has an excellent catalytic activity and is reusable in an oxidation reaction of an organic compound. The present invention has been made based on these findings.

Specifically, the present invention provides, in an embodiment, a method for recovering an oxoacid catalyst, where the oxoacid catalyst has been used in a reaction for oxidizing an organic compound with hydrogen peroxide in an aqueous/organic solvent two-phase reaction system. The method includes Step 1. In Step 1, the pH in the reaction system is adjusted to 5.0 or higher so as to transfer the oxoacid catalyst to the aqueous phase, and the organic phase is removed.

The method for recovering an oxoacid catalyst may further include Step 2 and Step 3. In Step 2, an organic solvent is added to the aqueous phase to give an aqueous/organic solvent two-phase reaction system. In Step 3, the pH in the reaction system is adjusted to lower than 5.0 and a phase transfer catalyst is added to the reaction system so as to transfer the oxoacid catalyst to the organic phase. The aqueous phase is then removed.

In the method for recovering an oxoacid catalyst, the oxoacid catalyst may include an oxoacid or a salt thereof, where the oxoacid includes at least one metal atom selected from the group consisting of tungsten, manganese, molybdenum, vanadium, niobium, tantalum, chromium, and rhenium.

The present invention further provides, in another embodiment, a method for producing an oxide. The method includes recovering an oxoacid catalyst by the method for recovering an oxoacid catalyst, where the oxoacid catalyst has been used in a reaction for oxidizing an organic compound with hydrogen peroxide in an aqueous/organic solvent two-phase reaction system. In the presence of the recovered oxoacid catalyst, an organic compound is oxidized with hydrogen peroxide to give the corresponding oxide.

Specifically, the present invention relates to followings.
[1] The present invention relates to a method for recovering an oxoacid catalyst, where the oxoacid catalyst has been used in a reaction for oxidizing an organic compound with hydrogen peroxide in an aqueous/organic solvent two-phase reaction system. The method includes Step 1. In Step 1, the pH in the reaction system is adjusted to 5.0 or higher so as to transfer the oxoacid catalyst to the aqueous phase, and the organic phase is then removed.
[2] The method for recovering an oxoacid catalyst according to [1] may further include Step 2 and Step 3. In Step 2, an organic solvent is added to the aqueous phase to give an aqueous/organic solvent two-phase reaction system. In Step 3, the pH in the reaction system is adjusted to lower than 5.0, and a phase transfer catalyst is added so as to transfer the oxoacid catalyst to the organic phase, and the aqueous phase is then removed.
[3] In the method for recovering an oxoacid catalyst according to one of [1] and [2], the oxoacid catalyst may include an oxoacid or a salt thereof, where the oxoacid contains at least one metal atom selected from the group consisting of tungsten, manganese, molybdenum, vanadium, niobium, tantalum, chromium, and rhenium.
[4] In the method for recovering an oxoacid catalyst according to one of [1] and [2], the oxoacid catalyst may include at least one compound or a salt thereof, where the at least one compound is selected from the group consisting of tungstic acid, manganic acid, molybdic acid, vanadic acid, tungstomolybdic acid, vanadomolybdic acid, vanadotungstic acid, manganotungstic acid, cobaltotungstic acid, manganomolybdotungstic acid, phosphotungstic acid, phosphomanganic acid, phosphomolybdic acid, phosphovanadic acid, silicotungstic acid, silicomolybdic acid, arsenotungstic acid, arsenomolybdic acid, phosphotungstomolybdic acid, phosphovanadomolybdic acid, and silicotungstomolybdic acid.
[5] In the method for recovering an oxoacid catalyst according to any one of [1] to [4], the oxoacid catalyst may include a metal-atom-containing oxoacid or a salt thereof, where the salt is selected from onium salts, alkali metal salts, alkaline earth metal salts, and transition metal salts.
[6] In the method for recovering an oxoacid catalyst according to any one of any one of [2] to [5], the phase transfer catalyst may include a quaternary ammonium salt represented by Formula (1): 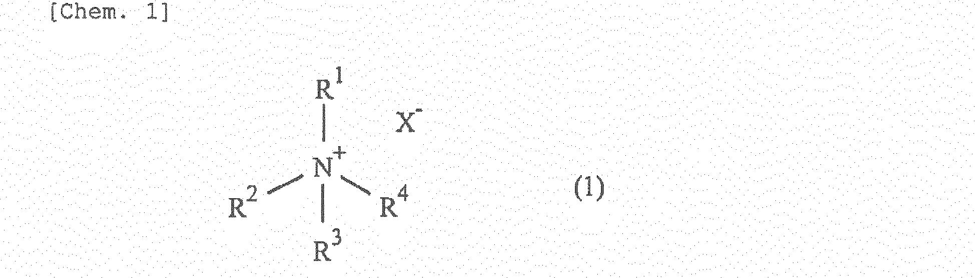 where R¹ to R⁴ each represent, identically or differently, an optionally substituted hydrocarbon group, where two or three selected from R¹ to R⁴ may be linked to each other to form a ring with the nitrogen cation (N⁺).
[7] In the method for recovering an oxoacid catalyst according to any one of [1] to [6], the organic compound may include at least one compound selected from the group consisting of straight or branched chain aliphatic hydrocarbons each containing a carbon-carbon double bond; compounds each containing a cycloalkene ring; and compounds each including two or more of these compounds bonded to each other with or without the medium of a linkage group.
[8] In the method for recovering an oxoacid catalyst according to any one of [1] to [6], the organic compound may include at least one of a compound represented by Formula (a-1) and a compound represented by Formula (a-2), where Formulae (a-1) and (a-2) are expressed as follows: 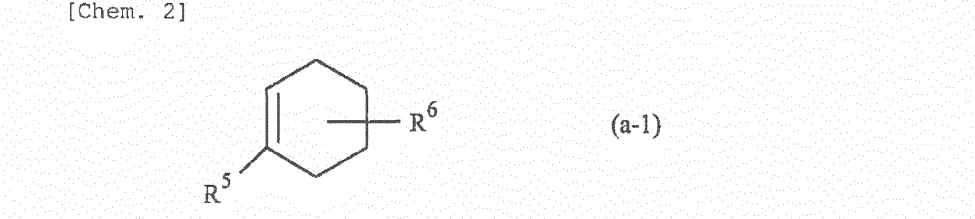 where R⁵ is selected from a hydrogen atom and an alkyl group; and R⁶ is selected from a hydrogen atom, an alkyl group, an alkenyl group, a hydroxy group, an alkoxy group, a carboxy group, and an alkoxycarbonyl group, 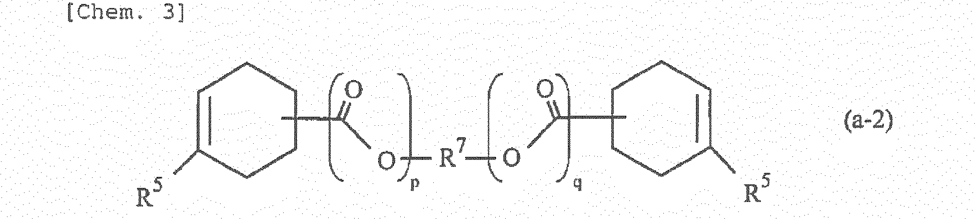 where R⁵ is, independently in each occurrence, selected from a hydrogen atom and an alkyl group; R⁷ is selected from a single bond and a straight or branched chain alkylene group; and p and q each represent, identically or differently, an integer of 0 or greater.
[9] In Step 1 of the method for recovering an oxoacid catalyst according to any one of [1] to [8], the organic phase may be removed after the passage of 0.5 to 20 hours following the pH adjustment.
[10] In Step 1 of the method for recovering an oxoacid catalyst according to any one of [1] to [9], the temperature in the reaction system may be adjusted in the range of from 30°C to 70°C.
[11] In Step 3 of the method for recovering an oxoacid catalyst according to any one of [2] to [10], the aqueous phase may be removed after the passage of 0.5 to 10 hours following the pH adjustment.
[12] In Step 3 of the method for recovering an oxoacid catalyst according to any one of [2] to [11], the temperature in the reaction system may be adjusted in the range of 50°C to 90°C.
[13] In the method for recovering an oxoacid catalyst according to any one of [1] to [12], 80 percent by weight or more of the entire oxoacid catalyst used in the reaction may be recovered.
[14] The present invention also relates to a method for producing an oxide. According to the method, an oxoacid catalyst is recovered by the method for recovering an oxoacid catalyst according to any one of [1] to [13], where the oxoacid catalyst has been used in a reaction for oxidizing an organic compound with hydrogen peroxide in an aqueous/organic solvent two-phase reaction system. In the presence of the recovered oxoacid catalyst, an organic compound is oxidized with hydrogen peroxide to yield the corresponding oxide.

### Advantageous Effects of Invention

The oxoacid catalyst recovery method according to the present invention has the configuration, thereby enables separation of the oxoacid catalyst from a reaction product by an easy procedure including pH control and separation operations alone, requires neither filtration treatment nor adsorption treatment, can avoid recovery rate reduction with these treatments, and can efficiently recover the oxoacid catalyst. In addition, the method can also purify and recover the oxoacid catalyst by an easy procedure including pH control and separation operations alone. The method is thereby very economically advantageous, can lighten the environmental load, and can significantly contribute to green chemistry. In general, a catalyst immobilized typically on a carrier suffers from deterioration in catalytic activity. However, the present invention eliminates the need of immobilizing the oxoacid catalyst typically on a carrier, can thereby prevent deterioration in catalytic activity with the immobilization of the catalyst typically on a carrier, and allows the oxoacid catalyst to maintain its catalytic activity at high level.

### Description of Embodiments

### Oxidation Reaction

The oxidation reaction in the present invention is a reaction for oxidizing an organic compound with hydrogen peroxide in the presence of an oxoacid catalyst in an aqueous/organic solvent two-phase reaction system.

### Oxoacid Catalyst

The oxoacid catalyst in the present invention is a compound that catalyzes a reaction for oxidizing an organic compound with hydrogen peroxide. Among such oxoacid catalysts, preferably used in the present invention is a metal-atom-containing oxoacid or a salt thereof. These are preferred for high partition coefficient toward the aqueous phase upon addition of hydrogen peroxide to the reaction system. The oxoacid may be a polyacid having a polynuclear complex structure such as Keggin structure or Dawson structure. Each of different oxoacid catalysts may be used alone or in combination.

The metal-atom-containing oxoacid is preferably an oxoacid containing at least one metal atom selected from the group consisting of tungsten (W), manganese (Mn), molybdenum (Mo), vanadium (V), niobium (Nb), tantalum (Ta), chromium (Cr), and rhenium (Re). The metal-atom-containing oxoacid preferably usable herein is exemplified by tungstic acid, manganic acid, molybdic acid, vanadic acid, tungstomolybdic acid, vanadomolybdic acid, vanadotungstic acid, manganotungstic acid, cobaltotungstic acid, and manganomolybdotungstic acid.

The salt of the metal-atom-containing oxoacid is exemplified by onium salts, alkali metal salts, alkaline earth metal salts, and transition metal salts of the above-exemplified metal-atom-containing oxoacids.

The metal-atom-containing oxoacid(s) may be used in combination with another oxoacid or a salt thereof. The "other oxoacid" refers to any of oxoacids excluding the metal-atom-containing oxoacids. The salt herein is exemplified by onium salts, alkali metal salts, alkaline earth metal salts, and transition metal salts. The other oxoacid or a salt thereof is exemplified by oxoacids each containing a phosphorus atom (P), a silicon atom (Si), or an arsenic atom (As), or salts of the oxoacids.

The phosphorus-containing oxoacids and salts thereof are exemplified by phosphoric acid, polyphosphoric acids (including pyrophosphoric acid and metaphosphoric acid), and (poly)phosphates. The (poly)phosphates are exemplified by alkali metal (poly)phosphates such as potassium phosphate and sodium phosphate; alkaline earth metal (poly)phosphates such as calcium phosphate; alkali metal hydrogen(poly)phosphates such as potassium hydrogenphosphate and sodium hydrogenphosphate; alkaline earth metal hydrogen(poly)phosphates such as calcium hydrogenphosphate; and aluminum (poly)phosphates (including a double salt of aluminum phosphate and aluminum pyrophosphate). The phosphorus-containing compounds further include diphosphorus pentoxide and other materials (or starting materials) to synthetize the phosphorus-containing compounds. Each of different phosphorus-containing compounds may be used alone or in combination.

The silicon-containing oxoacids and salts thereof are exemplified by silicic acids such as orthosilicic acid and metasilicic acid. The arsenic-containing oxoacids and salts thereof are exemplified by arsenic acid and arsenious acid.

The metal-atom-containing oxoacid may form a condensate with the other oxoacid. The condensate is exemplified by phosphotungstic acid, phosphomanganic acid, phosphomolybdic acid, phosphovanadic acid, silicotungstic acid, silicomolybdic acid, arsenotungstic acid, arsenomolybdic acid, phosphotungstomolybdic acid, phosphovanadomolybdic acid, and silicotungstomolybdic acid. The condensate may also be a heteropolyacid having a polynuclear complex structure such as a Keggin structure or a Dawson structure.

Among them, the oxoacid catalyst for use in the present invention preferably includes an oxoacid containing at least one metal atom selected from the group consisting of tungsten, manganese, and vanadium, or a salt of the oxoacid in combination with a phosphorus-containing oxoacid or a salt of the phosphorus-containing oxoacid.

### Phase Transfer Catalyst

The oxoacid catalyst in the present invention is preferably used in combination with a phase transfer catalyst. The oxoacid catalyst, when used in combination with the phase transfer catalyst, can have better catalytic efficiency. The phase transfer catalyst for use herein may be selected from known or common quaternary ammonium salts.

The quaternary ammonium salts are exemplified by a compound represented by Formula (1):

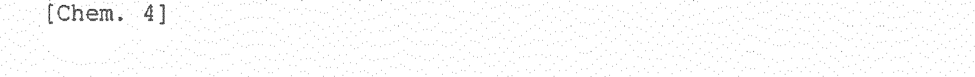

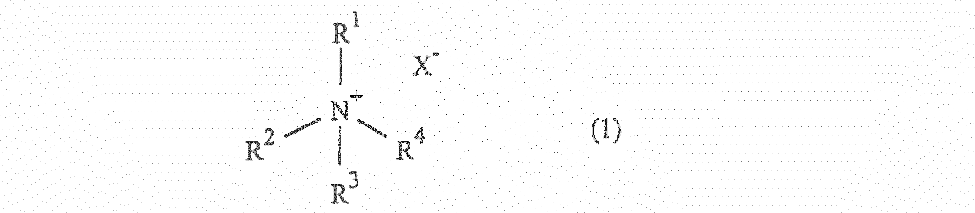

In Formula (1), R¹ to R⁴ each represent, identically or differently, a hydrocarbon group. The hydrocarbon group is exemplified by aliphatic hydrocarbon groups, alicyclic hydrocarbon groups, aromatic hydrocarbon groups, and groups each including two or more of them bonded to each other. The hydrocarbon group may have one or more substituents. Two or three selected from R¹ to R⁴ may be linked to each other to form a ring with the nitrogen cation (N⁺).

Of the aliphatic hydrocarbon groups, preferred are saturated aliphatic hydrocarbon groups which are exemplified by straight or branched chain C₁-C₂₀ alkyl groups such as methyl, ethyl, propyl, isopropyl, butyl, hexyl, octyl, isooctyl, decyl, dodecyl, and octadecyl (i.e., stearyl) groups.

The alicyclic hydrocarbon groups are exemplified by C₃-C₁₂ cycloalkyl groups such as cyclopropyl, cyclobutyl, cyclopentyl, cyclohexyl, and cyclododecyl groups.

The aromatic hydrocarbon groups are exemplified by C₆-C₁₄ aryl groups such as phenyl and naphthyl groups, of which C₆-C₁₀ aryl groups are preferred.

The groups each including an aliphatic hydrocarbon group and an alicyclic hydrocarbon group bonded to each other are exemplified by (C₃-C₁₂) cycloalkyl)-substituted C₁-C₂₀ alkyl groups such as cyclohexylmethyl group; and (C₁-C₂₀ alkyl)-substituted C₃-C₁₂ cycloalkyl groups such as methylcyclohexyl group. The groups each including an aliphatic hydrocarbon group and an aromatic hydrocarbon group bonded to each other are exemplified by C₇-C₁₈ aralkyl groups such as benzyl and phenethyl groups, of which C₇-C₁₀ aralkyl groups are preferred; and (C₁-C₄ alkyl)-substituted aryl groups such as tolyl group.

The substituents which the hydrocarbon groups as R¹ to R⁴ may have are exemplified by halogen atoms such as fluorine, chlorine, and bromine atoms; hydroxy group; C₁-C₆ alkoxy groups such as methoxy, ethoxy, propoxy, isopropyloxy, butoxy, and isobutyloxy groups; C₆-C₁₄ aryloxy groups optionally being substituted on the aromatic ring with one or more substituents (e.g., C₁-C₄ alkyl groups, halogen atoms, and C₁-C₄ alkoxy groups), such as phenoxy, tolyloxy, and naphthyloxy groups; C₇-C₁₈ aralkyloxy groups such as benzyloxy and phenethyloxy groups; C₁-C₁₂ acyloxy groups such as acetyloxy, propionyloxy, and benzoyloxy groups; carboxy group; C₁-C₁₆ alkoxy-carbonyl groups such as methoxycarbonyl, ethoxycarbonyl, propoxycarbonyl, and butoxycarbonyl groups; C₆-C₁₉ aryloxy-carbonyl groups such as phenoxycarbonyl, tolyloxycarbonyl, and naphthyloxycarbonyl groups; C₇-C₁₈ aralkyloxy-carbonyl groups such as benzyloxycarbonyl group; amino group; substituted amino groups including mono- or di-(C₁-C₆ alkyl)amino groups such as methylamino, ethylamino, dimethylamino, and diethylamino groups, and C₁-C₁₁ acylamino groups such as acetylamino, propionylamino, and benzoylamino groups; epoxy-containing groups such as glycidyloxy group; oxetanyl-containing groups such as ethyloxetanyloxy group; acyl groups such as acetyl, propionyl, and benzoyl groups; oxo group; and groups each including two or more of them bonded to each other as needed via a C₁-C₆ alkylene group.

Two or more selected from R¹ to R⁴ may be linked to each other to form a ring with the nitrogen cation (N⁺). The ring is exemplified by pyrrole ring, pyrrolidine ring, pyridine ring, and piperidine ring. The ring may have one or more substituents. The substituents are exemplified as with the substituents which the hydrocarbon groups as R¹ to R⁴ may have.

In Formula (1), X⁻ is a counter anion (counter ion; monovalent anion) with respect to the ammonium cation (quaternary ammonium ion) in the quaternary ammonium salt represented by Formula (1). X⁻ is exemplified by conjugate bases of Broensted acids, such as halide ions (e.g., fluoride, chloride, and iodide ions), hydrogensulfate ion, nitrate ion, hydrogencarbonate ion, perchlorate ion, tetrafluoroborate ion, hexafluorophosphite ion, methanesulfonate ion, trifluoromethanesulfonate ion, toluenesulfonate ion, formate ion, acetate ion, trifluoroacetate ion, propionate ion, benzoate ion, hydroxide ion, and alkoxide ions (e.g., methoxide ion and ethoxide ion). Among them, halide ions are preferred in the present invention.

Specifically, the quaternary ammonium salts are exemplified by trioctylmethylammonium chloride, trioctylethylammonium chloride, dilauryldimethylammonium chloride, lauryltrimethylammonium chloride, stearyltrimethylammonium chloride, lauryldimethylbenzylammonium chloride, stearyldimethylbenzylammonium chloride, didecyldimethylammonium chloride, tetrabutylammonium chloride, benzyltrimethylammonium chloride, benzyltriethylammonium chloride, trioctylmethylammonium bromide, trioctylethylammonium bromide, dilauryldimethylammonium bromide, lauryltrimethylammonium bromide, stearyltrimethylammonium bromide, lauryldimethylbenzylammonium bromide, stearyldimethylbenzylammonium bromide, didecyldimethylammonium bromide, tetrabutylammonium bromide, benzyltrimethylammonium bromide, benzyltriethylammonium bromide, trioctylmethylammonium iodide, trioctylethylammonium iodide, dilauryldimethylammonium iodide, lauryltrimethylammonium iodide, stearyltrimethylammonium iodide, lauryldimethylbenzylammonium iodide, stearyldimethylbenzylammonium iodide, didecyldimethylammonium iodide, tetrabutylammonium iodide, benzyltrimethylammonium iodide, benzyltriethylammonium iodide, trioctylmethylammonium hydrogenphosphate, trioctylethylammonium hydrogenphosphate, dilauryldimethylammonium hydrogenphosphate, lauryltrimethylammonium hydrogenphosphate, stearyltrimethylammonium hydrogenphosphate, lauryldimethylbenzylammonium hydrogenphosphate, stearyldimethylbenzylammonium hydrogenphosphate, didecyldimethylammonium hydrogenphosphate, tetrabutylammonium hydrogenphosphate, benzyltrimethylammonium hydrogenphosphate, benzyltriethylammonium hydrogenphosphate, trioctylmethylammonium hydrogensulfate, trioctylethylammonium hydrogensulfate, dilauryldimethylammonium hydrogensulfate, lauryltrimethylammonium hydrogensulfate, stearyltrimethylammonium hydrogensulfate, lauryldimethylbenzylammonium hydrogensulfate, stearyldimethylbenzylammonium hydrogensulfate, didecyldimethylammonium hydrogensulfate, tetrabutylammonium hydrogensulfate, benzyltrimethylammonium hydrogensulfate, benzyltriethylammonium hydrogensulfate, 1-methylpyridinium chloride, 1-methylpyridinium bromide, 1-ethylpyridinium chloride, 1-ethylpyridinium bromide, 1-n-butylpyridinium chloride, 1-n-butylpyridinium bromide, 1-n-hexylpyridinium chloride, 1-n-hexylpyridinium bromide, 1-n-octylpyridinium bromide, 1-n-dodecylpyridinium chloride, 1-dodecyl(2-methylpyridinium) chloride, 1-dodecyl(3-methylpyridinium) chloride, 1-dodecyl(4-methylpyridinium) chloride, 1-n-dodecylpyridinium bromide, 1-n-cetylpyridinium chloride, 1-n-cetylpyridinium bromide, 1-phenylpyridinium chloride, 1-phenylpyridinium bromide, 1-benzylpyridinium chloride, and 1-benzylpyridinium bromide. Each of them may be used alone or in combination.

The phase transfer catalyst may be used in an amount of typically about 0.01 to about 2.0 mol, preferably 0.05 to 1.0 mol, and particularly preferably 0.1 to 0.5 mol, per 1 mol of the oxoacid catalyst (an amount corresponding to 1 mol of the oxoacid catalyst in the case of a precursor compound).

### Hydrogen Peroxide

Hydrogen peroxide (or an aqueous hydrogen peroxide solution) for use as an oxidizing agent may be prepared synthetically by a common procedure, or may be available as a commercial product. The aqueous hydrogen peroxide solution, when used, may have a hydrogen peroxide concentration of preferably 5 to 80 percent by weight, particularly preferably 20 to 70 percent by weight, and most preferably 25 to 65 percent by weight. This is preferred from the viewpoint of handleability.

The hydrogen peroxide (substantially added hydrogen peroxide) may be used in an amount not critical, but typically about 0.1 to about 10 mol, preferably 0.2 to 5 mol, and particularly preferably 0.5 to 2 mol, per 1 mol of double bond contained in the after-mentioned compound containing a carbon-carbon double bond.

### Organic Compound

The organic compound for use in the oxidation reaction in the present invention may be any compound that is oxidized with hydrogen peroxide. Such compound is exemplified by compounds each containing at least one carbon-carbon double bond (hereinafter also referred to as "olefin(s)"), alcohols, and ketones. An olefin, when oxidized with hydrogen peroxide, generally forms a corresponding epoxy compound as a corresponding oxide (or a reaction product), as a result of epoxidation of the carbon-carbon double bond. The olefin also forms a diol under some conditions. A primary alcohol, when oxidized with hydrogen peroxide, forms, for example, an aldehyde and/or a carboxylic acid. A secondary alcohol, when oxidized with hydrogen peroxide, forms, for example, a ketone and/or a carboxylic acid. Ketones, when oxidized with hydrogen peroxide, undergo Baeyer-Villiger oxidation. As a result, a chain ketone forms an ester upon oxidation; and a cyclic ketone forms a lactone upon oxidation. Of such oxidation reactions with hydrogen peroxide, most representative oxidation reactions are olefin oxidation reactions, of which an epoxidation reaction is typified. The olefin epoxidation (epoxidation of olefin carbon-carbon double bond) reaction will be illustrated in detail below. It should be noted, however, that the oxoacid catalyst recovery method according to the present invention can be applied not only to this reaction, but also to any of the oxidation reactions.

The "olefin" is a compound containing at least one carbon-carbon double bond in molecule (per molecule). Exemplary olefins include (i) straight or branched chain aliphatic hydrocarbons containing a carbon-carbon double bond; (ii) compounds containing a cycloalkene ring (including a cycloalkapolyene ring such as a cycloalkadiene ring); and (iii) compounds each including one or more of them bonded to each other with or without the medium of a linkage group. These compounds may each have one or more substituents.

The straight or branched chain aliphatic hydrocarbons (i) containing a carbon-carbon double bond are exemplified by C₂-C₄₀ alkenes such as ethylene, propene, 1-butene, 2-butene, 1-pentene, 2-pentene, 1-hexene, 2-hexene, 2,3-dimethyl-2-butene, 3-hexene, 1-heptene, 2-heptene, 1-octene, 2-octene, 3-octene, 2-methyl-2-butene, 1-nonene, 2-nonene, decene, undecene, dodecene, tetradecene, hexadecene, and octadecene, of which C₂-C₃₀ alkenes are preferred, and C₂-C₂₀ alkenes are particularly preferred; C₄-C₄₀ alkadienes such as butadiene, isoprene, 1,5-hexadiene, 1,6-heptadiene, 1,7-octadiene, decadienes, undecadienes, and dodecadienes, of which C₄-C₃₀ alkadienes are preferred, and C₄-C₂₀ alkadienes are particularly preferred; C₆-C₃₀ alkatrienes such as undecatrienes and dodecatrienes, of which C₆-C₂₀ alkatrienes are preferred. Each of them may be used alone or in combination.

The straight or branched chain aliphatic hydrocarbons containing a carbon-carbon double bond may each have one or more substituents. The substituents are exemplified by aromatic hydrocarbon groups including C₆-C₁₀ aryl groups such as phenyl group; hydroxy group; halogen atoms such as fluorine, chlorine, and bromine atoms; mercapto group; alkoxy groups including C₁-C₁₀ alkoxy groups such as methoxy, ethoxy, propoxy, butoxy, and t-butoxy groups; C₁-C₆ haloalkoxy groups; alkylthio groups including C₁-C₁₀ alkylthio groups such as methylthio and ethylthio groups; carboxy group; alkoxycarbonyl groups including C₁-C₁₀ alkoxycarbonyl groups such as methoxycarbonyl and ethoxycarbonyl groups; acyl groups including C₂-C₁₀ acyl groups such as acetyl, propionyl, and trifluoroacetyl groups; acyloxy groups including C₁-C₁₀ acyloxy groups such as acetoxy, propionyloxy, and trifluoroacetoxy groups; amino group; substituted amino groups including mono- or di-(C₁-C₆ alkyl)amino groups such as methylamino, ethylamino, diethylamino, and diethylamino groups, and C₁-C₁₁ acylamino groups such as acetylamino, propionylamino, and benzoylamino groups; nitro group; cyano group; heterocyclic groups including nitrogen-containing heterocyclic groups such as pyridyl group. The number and substituted position(s) of the substituent(s) are not limited.

The substituted straight or branched chain aliphatic hydrocarbons are exemplified by phenylethylene (i.e., styrene), 1-phenylpropene, 2-phenyl-1-butene, 1-phenyl-1,3-butadiene, and 1-phenyl-1,3-pentadiene.

The compounds (ii) containing a cycloalkene ring (including a cycloalkapolyene ring such as a cycloalkadiene ring) are exemplified by C₃-C₂₀ cycloalkenes such as cyclopropene, cyclobutene, cyclopentene, cyclohexene, cycloheptene, cyclooctene, cyclononene, cyclodecene, cycloundecene, and cyclododecene, of which C₄-C₁₄ cycloalkenes are preferred, C₅-C₁₀ cycloalkenes are particularly preferred, and C₅-C₆ cycloalkenes are most preferred; C₅-C₂₀ cycloalkadienes such as cyclopentadiene, 1,3-cyclohexadiene, 1,4-cyclohexadiene, 1,3-cycloheptadiene, 1,4-cycloheptadiene, 1,5-cyclooctadiene, and cyclodecadienes, of which C₅-C₁₄ cycloalkadienes are preferred, and C₅-C₁₀ cycloalkadienes are particularly preferred; and C₇-C₂₀ cycloalkatrienes such as cyclooctatrienes. Each of them may be used alone or in combination.

These compounds may each have one or more substituents on the cycloalkene rings. The substituents are exemplified as with the substituents which the straight or branched chain aliphatic hydrocarbons containing a carbon-carbon double bond may have; as well as alkyl groups including C₁-C₁₀ alkyl groups such as methyl, ethyl, isopropyl, butyl, isobutyl, and t-butyl groups; C₁-C₁₀ haloalkyl groups; and alkenyl groups including C₂-C₁₀ alkenyl groups such as vinyl, allyl, propenyl, isopropenyl, and butenyl groups. The number and substituted position(s) of the substituent(s) are not limited.

The linkage group is exemplified by groups each including at least one selected from an alkylene group, an arylene group, a carbonyl bond, an ester bond, an amide bond, an ether bond, and a urethane bond. The alkylene group is exemplified by C₁-C₂₀ alkylene groups such as ethylene, propylene, trimethylene, tetramethylene, and 2-methylbutane-1,3-diyl group; and C₄-C₁₀ cycloalkylene groups such as 1,4-cyclohexylene group. Such exemplary alkylene groups also include alkylidene groups. The arylene group is exemplified by C₆-C₁₀ arylene groups such as phenylene and naphthalenediyl groups.

The olefin may contain carbon atoms in a number of typically about 2 to about 40, preferably 6 or more (e.g., 6 to 30), more preferably 6 to 25, particularly preferably 6 to 20, and most preferably 7 to 20. When the olefin contains a substituent(s) and/or a linkage group, the number of carbons is the total sum of the number of carbons in the olefin and the number of carbons contained the substituent(s) and/or linkage group. When the olefin contains both a substituent(s) and a linkage group, the number of carbons is the total sum of the number of carbons in the olefin and the number of carbons in the substituent(s) and linkage group.

Representative examples of the olefin include a compound represented by Formula (a-1) and a compound represented by Formula (a-2): 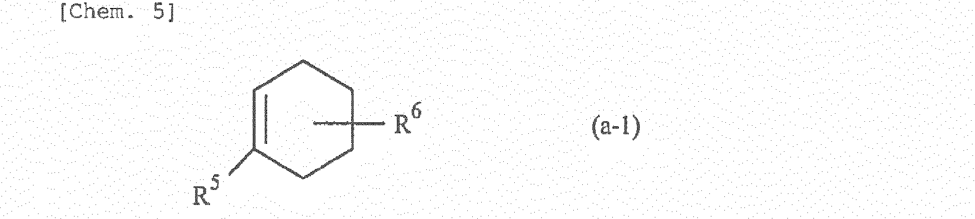 where R⁵ is selected from a hydrogen atom and an alkyl group; and R⁶ is selected from a hydrogen atom, an alkyl group, an alkenyl group, a hydroxy group, an alkoxy group, a carboxy group, and an alkoxycarbonyl group, 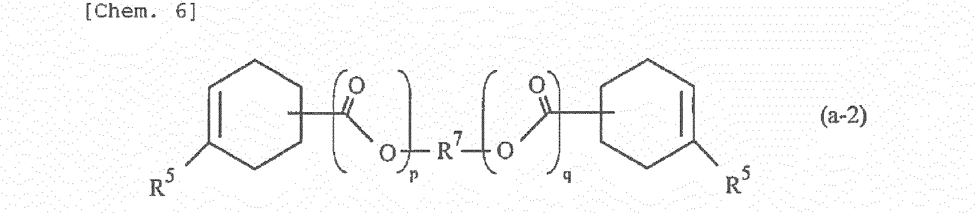 where R⁵ is, independently in each occurrence, selected from a hydrogen atom and an alkyl group; R⁷ is selected from a single bond and a straight or branched chain alkylene group; and p and q each represent, identically or differently, an integer of 0 or greater. The compound represented by Formula (a-2), in which p and q are 0 and R⁷ is a single bond, has a structure including two cyclohexene rings bonded via a single bond.

The alkyl groups as R⁵ and R⁶ are exemplified by straight or branched chain C₁-C₄ alkyl groups such as methyl, ethyl, butyl, and isobutyl groups.

As R⁶, the alkenyl group is exemplified by C₂-C₁₀ alkenyl groups such as vinyl, allyl, propenyl, isopropenyl, and butenyl groups. The alkoxy group is exemplified by C₁-C₁₀ alkoxy groups such as methoxy, ethoxy, propoxy, butoxy, and t-butoxy groups. The alkoxycarbonyl group is exemplified by C₁-C₁₀ alkoxycarbonyl groups such as methoxycarbonyl and ethoxycarbonyl groups.

The straight or branched chain alkylene group (including an alkylidene group) as R⁷ is exemplified by straight or branched chain C₂-C₂₀ alkylene groups (or alkylidene groups) such as methylene, ethylene, propylene, and 2,2-dimethylpropane-1,3-diyl groups.

The numbers p and q each represent, identically or differently, an integer of 0 or greater and are particularly preferably both 1.

The compounds represented by Formulae (a-1) and (a-2) are exemplified by compounds represented by Formulae (b-1) to (b-9):

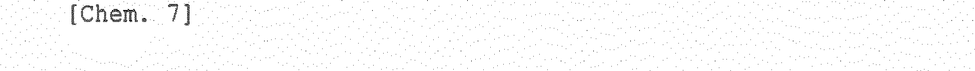

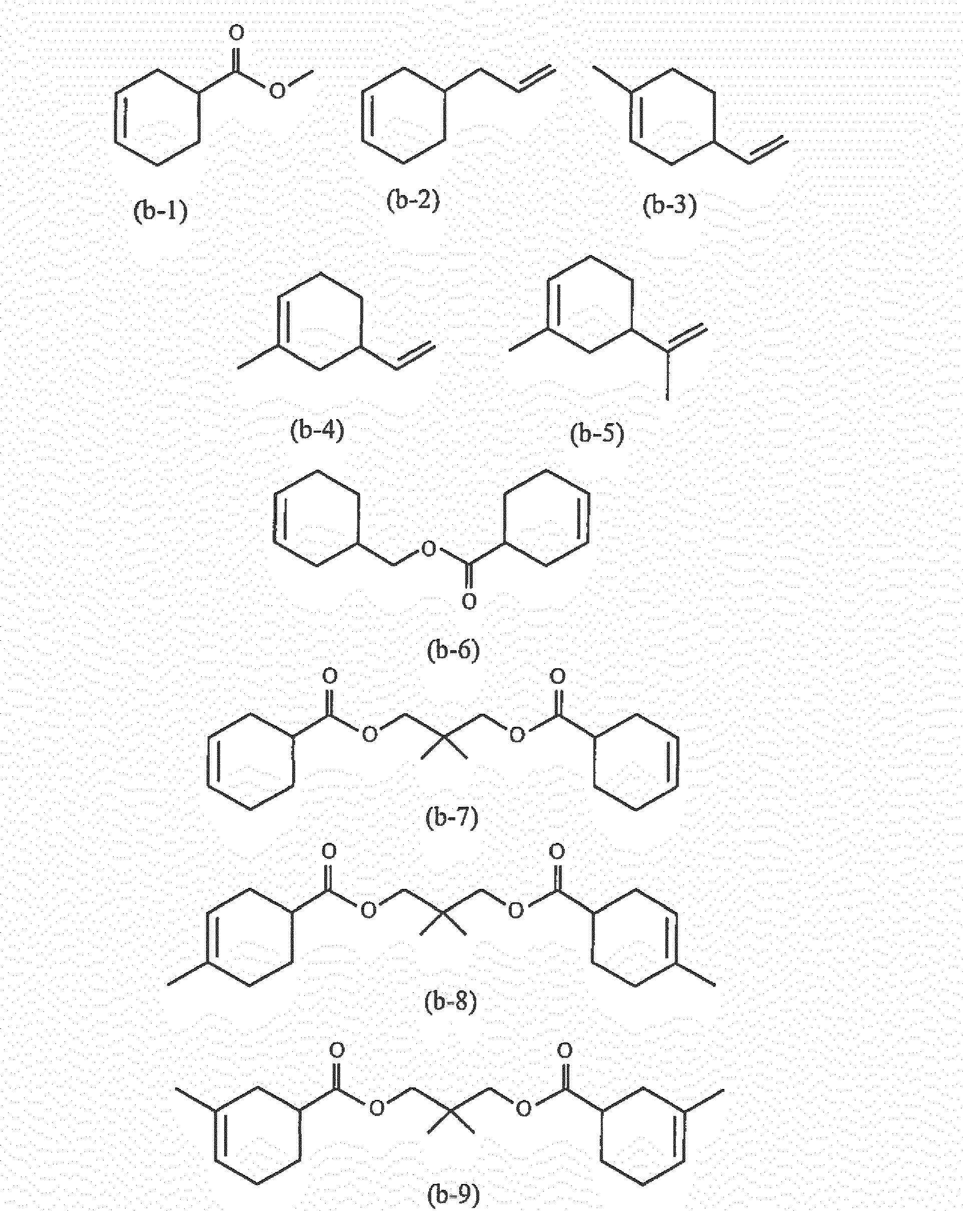

Typically, the compound represented by Formula (b-3), when used as the organic compound, gives a diepoxy compound and a monoepoxy compound respectively represented by Formula (c-3-1) and Formula (c-3-2) below.

The compound represented by Formula (b-6), when used as the organic compound, gives an epoxy compound represented by Formula (c-6) (3,4-epoxycyclohexylmethyl (3,4-epoxy)cyclohexanecarboxylate).

The compound represented by Formula (b-8), when used as the organic compound, gives an epoxy compound represented by Formula (c-8).

The compound represented by Formula (b-9), when used as the organic compound, gives an epoxy compound represented by Formula (c-9):

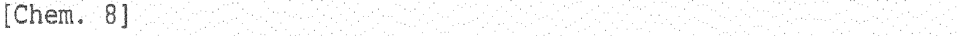

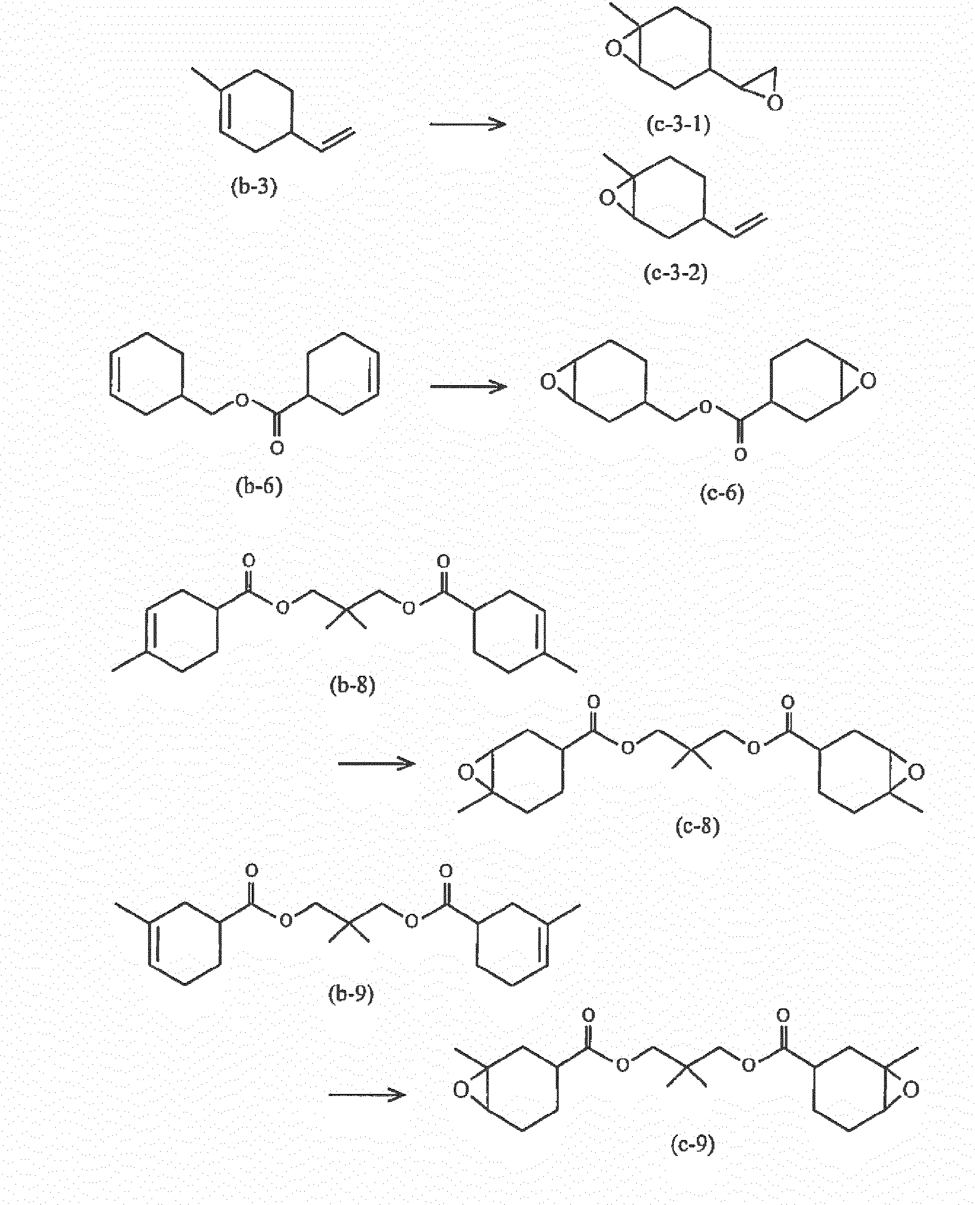

The oxidation reaction in the present invention is performed in an aqueous/organic solvent two-phase reaction system. The organic solvent is not limited, as long as capable of separating from an aqueous solvent, and can be selected as appropriate according to the type of the organic compound (e.g., an olefin) to be oxidized. The organic solvent is exemplified by C₃-C₁₀ cycloalkanols such as cyclopropanol and cyclohexanol; chain ethers such as dimethyl ether and diethyl ether; ketones such as methyl ethyl ketone, methyl isobutyl ketone, cyclopentanone, and cyclohexanone; esters including chain esters such as ethyl acetate, butyl acetate, methyl lactate, and ethyl lactate; hydrocarbons; halogenated hydrocarbons such as chloroform, methylene chloride, and chlorobenzene; and phenols. The hydrocarbons are exemplified by aliphatic hydrocarbons such as pentane, hexane, and heptane; alicyclic hydrocarbons such as cyclohexane and methylcyclohexane; and aromatic hydrocarbons such as toluene, xylenes, and ethylbenzene. Each of the organic solvents may be used alone or in combination. Of these organic solvents, preferred from the viewpoint of reaction efficiency are aromatic hydrocarbons, halogenated hydrocarbons, and alicyclic hydrocarbons, of which chlorobenzene, toluene, and cyclohexane are particularly preferred.

The proportions of the aqueous solvent and the organic solvent may be such proportions that the ratio (weight ratio) of the former to the latter is typically about 0.005 to about 2.0, preferably 0.01 to 1.0, and particularly preferably 0.03 to 0.75. The aqueous solvent may be used in an amount of typically about 0.01 to about 10 parts by weight, preferably 0.05 to 5 parts by weight, and particularly preferably 0.1 to 2 parts by weight, per 1 part by weight of the organic compound (e.g., an olefin).

The oxidation reaction in the present invention may be performed typically by adding hydrogen peroxide dropwise to a reactor into which the organic compound, phase transfer catalyst, oxoacid catalyst, and solvents have been charged. The reaction (or the dropwise addition of hydrogen peroxide) may be performed for a time of typically about 0.1 to about 12 hours. After the completion of dropwise addition, the reaction mixture may be aged for a period of about 0.5 to about 20 hours.

The reaction system during the oxidation reaction preferably has a pH adjusted within the range of about 3.0 to about 7.5 (more preferably 3.5 to 7.0). The pH adjustment may be performed using a phosphate. The phosphate is exemplified by disodium hydrogenphosphate dodecahydrate and sodium dihydrogenphosphate dihydrate, each of which may be used alone or in combination.

The reaction (or the dropwise addition of hydrogen peroxide) may be performed at a temperature (or a temperature in the reaction system) of typically about 30°C to about 70°C. The reaction may be performed under normal atmospheric pressure, under reduced pressure, or under pressure (under a load). The reaction may be performed in any atmosphere not limited, as long as not adversely affecting the reaction, such as air atmosphere, nitrogen atmosphere, or argon atmosphere.

### Oxoacid Catalyst Recovery Method

The oxoacid catalyst recovery method according to the present invention is a method for recovering an oxoacid catalyst, where the oxoacid catalyst has been used in a reaction for oxidizing an organic compound with hydrogen peroxide in an aqueous/organic solvent two-phase system. The method includes Step 1. In Step 1, the pH in the reaction system is adjusted to 5.0 or higher so as to transfer the oxoacid catalyst to the aqueous phase, and the organic phase is then removed.

The pH may be adjusted using a strong base such as sodium hydroxide, potassium hydroxide, calcium hydroxide, or tetramethylammonium hydroxide. Each of them may be used alone or in combination.

Optimum reaction conditions including optimum pH may vary depending on the substrate. In this connection, when the pH in the reaction system is lower than 5, the oxoacid catalyst is present both in the aqueous phase and in the organic phase. When the pH in the reaction system is adjusted to 5.0 or higher (preferably 5.0 to 13.0, and particularly preferably 9.0 to 12.0), the oxoacid catalyst can be converted into a water-soluble salt, and this enables transfer of 85 percent by weight or more (preferably 90 percent by weight or more) of the entire oxoacid catalyst in the reaction system to the aqueous phase.

The reaction system after the pH adjustment is preferably stirred for typically about 0.5 to about 20 hours, and preferably 1 to 10 hours, before the removal of the organic phase. This procedure is preferred so that 85 percent by weight or more (preferably 90 percent by weight or more) of the entire oxoacid catalyst in the reaction system can be recovered into the aqueous phase. The organic phase removal, if performed within an excessively short time after the pH adjustment, may readily cause a lower recovery rate of the oxoacid catalyst.

The reaction system upon transfer of the oxoacid catalyst to the aqueous phase may have a temperature of typically about 30°C to about 70°C. The reaction (transfer), if performed at a temperature higher than the range, may readily cause the reaction product to decompose. In contrast, the transfer of the oxoacid catalyst, if performed at a reaction temperature lower than the range, may often require a long time and may thereby cause lower working efficiency. The transfer of the oxoacid catalyst to the aqueous phase may be performed in any atmosphere of the reaction system, as long as not adversely affecting the reaction, such as air atmosphere, nitrogen atmosphere, or argon atmosphere.

The reaction product is present in the organic phase regardless of the pH change in the reaction system. Thus, when the pH in the reaction system is within the range so as to transfer the oxoacid catalyst to the aqueous phase, and thereafter the the organic phase is removed, the oxoacid catalyst can be separated from the reaction product and can be recovered in the aqueous phase. The removal of the organic phase enables removal of impurities from the reaction system, where the impurities have solubility in the organic solvent. Thus, the oxoacid catalyst can be recovered in the aqueous phase, where the oxoacid catalyst is recovered as a purified catalyst that contains substantially no impurities having solubility in the organic solvent.

The oxoacid catalyst recovery method according to the present invention preferably further includes Step 2 and Step 3. This is preferred for recovering the oxoacid catalyst as a purified catalyst that contains substantially no impurities having solubility in the organic solvent and substantially no impurities having solubility in water. In Step 2, an organic solvent is added to the aqueous phase to give an aqueous/organic solvent two-phase reaction system. In Step 3, the pH in the reaction system is adjusted to lower than 5.0, and a phase transfer catalyst is added to the system so as to transfer the oxoacid catalyst to the organic phase, and the aqueous phase is thereafter removed.

In Step 2, an organic solvent identical to the separated and removed organic phase is preferably added to give the aqueous/organic solvent two-phase system.

In Step 3, the pH in the reaction system is adjusted to lower than 5.0, preferably 4.8 or less, more preferably lower than 4.8, and particularly preferably 2.0 to 4.5, and a phase transfer catalyst is added so as to transfer the oxoacid catalyst to the organic phase. The aqueous phase is then removed. The removal of the aqueous phase enables the removal of impurities having solubility in water from the reaction system. Thus, the oxoacid catalyst can be recovered in the organic phase, where the oxoacid catalyst is obtained as a purified catalyst that contains substantially no impurities having solubility in the organic solvent and substantially no impurities having solubility in water.

The pH adjuster for use in Step 3 is exemplified by acids such as hydrochloric acid, sulfuric acid, phosphoric acid, and acetic acid. Each of them may be used alone or in combination.

The reaction system after the pH adjustment in Step 3 is preferably stirred for typically about 0.5 to about 10 hours, and preferably 1 to 5 hours, before the removal of the aqueous phase. The aqueous phase removal, if performed within an excessively short time after the pH adjustment, may readily cause a lower recovery rate of the oxoacid catalyst.

The reaction system in Step 3 may have a temperature of typically about 50°C to about 90°C. The reaction temperature, if set higher than the range, may readily fail to give advantageous effects such as promotion of working efficiency and may often become uneconomical. In contrast, the reaction temperature, if being lower than the range, may readily cause a long time for the oxoacid catalyst transfer and may often cause lower working efficiency. The reaction system in Step 3 may have any atmosphere not limited, as long as not adversely affecting the reaction, such as air atmosphere, nitrogen atmosphere, or argon atmosphere.

The oxoacid catalyst recovery method according to the present invention enables recovery and reuse of the oxoacid catalyst used in the reaction by easy operations including pH adjustment and separating operations alone, where the oxoacid catalyst can be recovered and reused in an amount of 80 percent by weight or more (preferably 83 percent by weight or more, and particularly preferably 85 percent by weight or more) of the entire oxoacid catalyst. The method is thereby very economically advantageous and can lighten the environmental load due to disposal of the oxoacid catalyst. In an embodiment, the method further includes Steps 2 and 3. The oxoacid catalyst recovered by the method according to this embodiment contains substantially no impurities having solubility in water and substantially no impurities having solubility in the organic solvent and can have excellent activity. The recovered organic acid, when reused in a reaction for oxidizing an organic compound with hydrogen peroxide in an aqueous/organic solvent two-phase system, can give a target compound in a high yield with a high selectivity.

### Oxide Production Method

The oxide production method according to the present invention includes recovering an oxoacid catalyst by the method for recovering an oxoacid catalyst, where the oxoacid catalyst has been used in a reaction for oxidizing an organic compound with hydrogen peroxide in an aqueous/organic solvent two-phase system. In the presence of the recovered (recycled) oxoacid catalyst, an organic compound is oxidized with hydrogen peroxide to give a corresponding oxide.

The resulting oxide obtained by oxidation of the organic compound with hydrogen peroxide is present in the organic phase. After the completion of the oxidation reaction, the pH in the reaction system is adjusted to 5.0 or higher so as to transfer the oxoacid catalyst to the aqueous phase. After this, the organic phase is separated or fractionated. The fractionated organic phase is subjected to a separation procedure to recover the oxide. The separation procedure is exemplified by concentration, distillation, extraction, or chromatography; and a separation procedure as any combination of them. According to the present invention, the oxoacid catalyst is used as not being immobilized typically on a carrier, but being highly dispersed. The method according to the present invention allows the oxoacid catalyst to avoid the deterioration in catalytic activity due to immobilization typically on a carrier, to exhibit excellent catalytic activity, and to give the oxide in a high yield. The method enables the separation between the oxoacid catalyst and the reaction product (oxide) without performing filtration treatment and adsorption treatment, can avoid the recovery rate reduction of the oxide due to the treatments, and can efficiently recover the oxide.

The oxide production method according to the present invention reuses the recovered oxoacid catalyst as mentioned above, is thereby very economically advantageous, and can lighten the environmental load with the disposal of the oxoacid catalyst. In addition, the oxide production method according to the present invention enables inexpensive and clean production of a corresponding oxide (e.g., an epoxy compound) from an organic compound.

### Examples

The present invention will be illustrated in further detail with reference to several examples below. It should be noted, however, that the examples are by no means intended to limit the scope of the present invention. The "amount of metallic tungsten" refers to an amount in terms of pure tungsten.

### Example 1

### Oxidation Reaction: Synthesis of 3,4-epoxycyclohexylmethyl (3,4-epoxy)cyclohexanecarboxylate

In a nitrogen atmosphere at room temperature, a 100-mL four-neck flask was charged with 3-cyclohexenylmethyl 3'-cyclohexenylcarboxylate (hereinafter also referred to as "CMCC") (10.00 g, 45.4 mmol), 69.6% trioctylmethylammonium chloride (0.296 g, 0.510 mmol), sodium tungstate dihydrate (0.834 g, 2.527 mmol), disodium hydrogenphosphate dodecahydrate (0.184 g, 0.514 mmol), 85% phosphoric acid (0.262 g, 2.27 mmol), toluene (30.0 g), and water (1.8 g), and the pH in the reaction system was thereby adjusted to 6.2. The reaction system with stirring was heated to 60°C and combined with a 35% aqueous hydrogen peroxide solution (13.06 g, 136.4 mmol) added dropwise over 20 minutes, followed by stirring for further 6 hours.

The amounts of tungsten contained in the organic phase and in the aqueous phase were determined by inductively coupled plasma (ICP) emission spectrometry to find that tungsten was present in the organic phase and in the aqueous phase respectively in amounts of 0.068 g and 0.397 g. The target compound (3,4-epoxycyclohexylmethyl (3,4-epoxy)cyclohexanecarboxylate) was found to be present in the organic phase in an amount of 10.20 g with a conversion of 98.3% and a selectivity of 90.4% in a yield of 88.9%.

### Catalyst Recovery: First recovery

In a nitrogen atmosphere, the system after the completion of the reaction was combined with a 5% aqueous sodium hydroxide solution (11.33 g) to adjust the pH in the reaction system to 11.7. The reaction system with stirring was heated to 40°C, followed by stirring for further 6 hours while maintaining the temperature at 90°C. The amounts of tungsten contained in the organic phase and in the aqueous phase were determined by inductively coupled plasma (ICP) emission spectrometry to find that tungsten was present in the organic phase and in the aqueous phase respectively in amounts of 0.069 g and 0.396 g. As a result of subsequent separation, the organic phase (38.2 g) and the aqueous phase (24.8 g) were recovered.

In a nitrogen atmosphere, the recovered aqueous phase was combined with 69.6% trioctylmethylammonium chloride (0.509 g, 0.879 mmol), toluene (25.5 g), and 85% phosphoric acid (2.18 g) to adjust the pH in the reaction system to 2.5. The reaction system with stirring was heated to 80°C, followed by stirring for further 4 hours while maintaining the temperature at 80°C. As a result of subsequent separation, the organic phase (25.9 g) and the aqueous phase (25.5 g) were recovered. The amounts of tungsten contained in the organic phase and in the aqueous phase were determined by inductively coupled plasma (ICP) emission spectrometry to find that tungsten was present in the organic phase and in the aqueous phase respectively in amounts of 0.388 g and 0.008 g. Thus, 83% of the initially charged amount of metallic tungsten could be recovered in the organic phase.

### Oxidation Reaction by Recycled Catalyst: First reaction

In a nitrogen atmosphere at room temperature, a 100-mL four-neck flask was charged with the organic phase (25.9 g) containing a tungstate (0.388 g in terms of pure tungsten) as recovered in the first catalyst recovery, CMCC (8.31 g, 37.7 mmol), disodium hydrogenphosphate dodecahydrate (1.49 g, 4.16 mmol), 85% phosphoric acid (0.213 g, 1.847 mmol), and water (1.5 g), and the pH in the reaction system was thereby adjusted to 5.9. The reaction system with stirring was heated to 60°C and combined with a 35% aqueous hydrogen peroxide solution (10.83 g, 111.5 mmol) added dropwise over 20 minutes, followed by stirring for further 6 hours. The amounts of tungsten contained in the organic phase and in the aqueous phase were determined by inductively coupled plasma (ICP) emission spectrometry to find that tungsten was present in the organic phase and in the aqueous phase respectively in amounts of 0.169 g and 0.219 g. The target compound (3,4-epoxycyclohexylmethyl (3,4-epoxy)cyclohexanecarboxylate) was found to be present in the organic phase in an amount of 8.71 g with a conversion of 98.4% and a selectivity of 93.0% in a yield of 91.5%.

### Catalyst Recovery: Second recovery

In a nitrogen atmosphere, the system after the completion of the first oxidation reaction by the recycled catalyst was combined with a 5% aqueous sodium hydroxide solution (14.57 g) to adjust the pH in the reaction system to 11.3. The reaction system with stirring was heated to 40°C, followed by stirring for further 6 hours while maintaining the temperature at 90°C. The amounts of tungsten contained in the organic phase and in the aqueous phase were determined by inductively coupled plasma (ICP) emission spectrometry to find that tungsten was present in the organic phase and in the aqueous phase respectively in amounts of 0.048 g and 0.340 g. As a result of subsequent separation, the organic phase (32.5 g) and the aqueous phase (27.4 g) were recovered.

In a nitrogen atmosphere, the recovered aqueous phase was combined with 69.6% trioctylmethylammonium chloride (0.471 g, 0.811 mmol), toluene (22.7 g), and 85% phosphoric acid (2.60 g), and the pH in the reaction system was thereby adjusted to 2.7. The reaction system with stirring was heated to 80°C, followed by stirring for further 4 hours while maintaining the temperature at 80°C. As a result of subsequent separation, the organic phase (23.0 g) and the aqueous phase (28.9 g) were recovered.

The amounts of tungsten contained in the organic phase and in the aqueous phase were determined by inductively coupled plasma (ICP) emission spectrometry to find that tungsten was present in the organic phase and in the aqueous phase respectively in amounts of 0.336 g and 0.004 g. Thus, 87% of the initially charged amount of metallic tungsten could be recovered in the organic phase.

### Oxidation Reaction by Recycled Catalyst: Second reaction

In a nitrogen atmosphere at room temperature, a 100-mL four-neck flask was charged with the organic phase (23.0 g) containing a tungstate (0.336 g in terms of pure tungsten) as recovered in the second catalyst recovery, CMCC (7.18 g, 32.6 mmol), disodium hydrogenphosphate dodecahydrate (1.29 g, 3.60 mmol), 85% phosphoric acid (0.185 g, 1.605 mmol), and water (1.3 g), and the pH in the reaction system was thereby adjusted to 5.7. The reaction system with stirring was heated to 60°C and combined with a 35% aqueous hydrogen peroxide solution (9.38 g, 96.5 mmol) added dropwise over 20 minutes, followed by stirring for further 6 hours.

The amounts of tungsten contained in the organic phase and in the aqueous phase were determined by inductively coupled plasma (ICP) emission spectrometry to find that tungsten was present in the organic phase and in the aqueous phase respectively in amounts of 0.156 g and 0.180 g. The target compound (3,4-epoxycyclohexylmethyl (3,4-epoxy)cyclohexanecarboxylate) was found to be present in the organic phase in an amount of 7.50 g with a conversion of 100.0% and a selectivity of 91.3% in a yield of 91.3%.

### Example 2

### Oxidation Reaction: Synthesis of 3, 4, 3', 4'-diepoxy)bicyclohexyl

In a nitrogen atmosphere at room temperature, a 100-mL four-neck flask was charged with bicyclohexyl-3,3'-diene (10.00 g, 61.6 mmol), 69.6% trioctylmethylammonium chloride (0.397 g, 0.684 mmol), sodium tungstate dihydrate (1.134 g, 3.438 mmol), disodium hydrogenphosphate dodecahydrate (0.246 g, 0.687 mmol), 85% phosphoric acid (0.358 g, 3.105 mmol), toluene (30.0 g), and water (1.8 g), and the pH in the reaction system was thereby adjusted to 6.2. The reaction system with stirring was heated to 55°C and combined with a 35% aqueous hydrogen peroxide solution (17.71 g, 182.3 mmol) added dropwise over 20 minutes, followed by stirring for further 6 hours. The amounts of tungsten contained in the organic phase and in the aqueous phase were determined by inductively coupled plasma (ICP) emission spectrometry to find that tungsten was present in the organic phase and in the aqueous phase respectively in amounts of 0.125 g and 0.476 g. The target compound ((3, 4, 3', 4'-diepoxy)bicyclohexyl) was found to be present in the organic phase in an amount of 11.02 g with a conversion of 100.0% and a selectivity of 91.9% in a yield of 91.9%.

### Catalyst Recovery: First recovery

In a nitrogen atmosphere, the system after the completion of the reaction was combined with a 5% aqueous sodium hydroxide solution (15.44 g) to adjust the pH in the reaction system to 11.4 and heated to 60°C with stirring, followed by stirring for further 2 hours while maintaining the temperature at 60°C. The amounts of tungsten contained in the organic phase and in the aqueous phase were determined by inductively coupled plasma (ICP) emission spectrometry to find that tungsten was present in the organic phase and in the aqueous phase respectively in amounts of 0.051 g and 0.581 g. As a result of subsequent separation, the organic phase (39.9 g) and the aqueous phase (34.6 g) were recovered.

In a nitrogen atmosphere, the recovered aqueous phase was combined with 69.6% trioctylmethylammonium chloride (0.728 g, 1.254 mmol), toluene (27.6 g), and 85% phosphoric acid (2.70 g) to adjust the pH in the reaction system to 3.1 and heated to 80°C with stirring, followed by stirring for further 4 hours while maintaining the temperature at 80°C. As a result of subsequent separation, the organic phase (28.4 g) and the aqueous phase (35.8 g) were recovered.

The amounts of tungsten contained in the organic phase and in the aqueous phase were determined by inductively coupled plasma (ICP) emission spectrometry to find that tungsten was present in the organic phase and in the aqueous phase respectively in amounts of 0.562 g and 0.019 g. Thus, 89% of the initially charged amount of metallic tungsten could be recovered in the organic phase.

### Oxidation Reaction by Recycled Catalyst: First reaction

In a nitrogen atmosphere at room temperature, a 100-mL four-neck flask was charged with the organic phase (28.4 g) containing a tungstate (0.562 g in terms of pure tungsten) as recovered in the first catalyst recovery, bicyclohexyl-3, 3'-diene (8.87 g, 54.7 mmol), disodium hydrogenphosphate dodecahydrate (2.16 g, 6.02 mmol), 85% phosphoric acid (0.338 g, 2.932 mmol), and water (1.6 g), and the pH in the reaction system was thereby adjusted to 5.6. The reaction system was heated to 55°C with stirring and combined with a 35% aqueous hydrogen peroxide solution (15.69 g, 161.5 mmol) added dropwise over 20 minutes, followed by stirring for further 6 hours. The amounts of tungsten contained in the organic phase and in the aqueous phase were determined by inductively coupled plasma (ICP) emission spectrometry to find that tungsten was present in the organic phase and in the aqueous phase respectively in amounts of 0.260 g and 0.302 g. The target compound ((3, 4, 3', 4'-diepoxy)bicyclohexyl) was found to be present in the organic phase in an amount of 10.35 g with a conversion of 100.0% and a selectivity of 97.5% in a yield of 97.5%.

### Catalyst Recovery: Second recovery

In a nitrogen atmosphere, the system after the completion of the first oxidation reaction by the recycled catalyst was combined with a 5% aqueous sodium hydroxide solution (19.62 g) to adjust the pH in the reaction system to 11.3 and heated to 60°C with stirring, followed by stirring for further 2 hours while maintaining the temperature at 60°C. The amounts of tungsten contained in the organic phase and in the aqueous phase were determined by inductively coupled plasma (ICP) emission spectrometry to find that tungsten was present in the organic phase and in the aqueous phase respectively in amounts of 0.051 g and 0.511 g. As a result of subsequent separation, the organic phase (34.6 g) and the aqueous phase (38.7 g) were recovered.

In a nitrogen atmosphere, the recovered aqueous phase was combined with 69.6% trioctylmethylammonium chloride (0.638 g, 1.099 mmol), toluene (24.1 g), and 85% phosphoric acid (2.70 g), and the pH in the reaction system was thereby adjusted to 3.4. The reaction system with stirring was heated to 80°C, followed by stirring for further 4 hours while maintaining the temperature at 80°C. As a result of subsequent separation, the organic phase (24.7 g) and the aqueous phase (40.2 g) were recovered.

The amounts of tungsten contained in the organic phase and in the aqueous phase were determined by inductively coupled plasma (ICP) emission spectrometry to find that tungsten was present in the organic phase and in the aqueous phase respectively in amounts of 0.500 g and 0.011 g. Thus, 89% of the initially charged amount of metallic tungsten could be recovered in the organic phase.

### Oxidation Reaction by Recycled Catalyst: Second reaction

In a nitrogen atmosphere at room temperature, a 100-mL four-neck flask was charged with the organic phase (24.7 g) containing a tungstate (0.500 g in terms of pure tungsten) as recovered in the second catalyst recovery, bicyclohexyl-3,3'-diene (7.88 g, 48.6 mmol), disodium hydrogenphosphate dodecahydrate (1.92 g, 5.36 mmol), 85% phosphoric acid (0.279 g, 2.420 mmol), and water (1.4 g), and the pH in the reaction system was thereby adjusted to 5.6. The reaction system with stirring was heated to 55°C and combined with a 35% aqueous hydrogen peroxide solution (13.95 g, 143.6 mmol) added dropwise over 20 minutes, followed by stirring for further 6 hours. The amounts of tungsten contained in the organic phase and in the aqueous phase were determined by inductively coupled plasma (ICP) emission spectrometry to find that tungsten was present in the organic phase and in the aqueous phase respectively in amounts of 0.210 g and 0.290 g. The target compound ((3,4,3',4'-diepoxy)bicyclohexyl) was found to be present in the organic phase in an amount of 9.40 g with a conversion of 100.0% and a selectivity of 99.4% in a yield of 99.4%.

### Catalyst Recovery: Third recovery

In a nitrogen atmosphere, the system after the completion of the second oxidation reaction by the recycled catalyst was combined with a 5% aqueous sodium hydroxide solution (15.05 g) to adjust the pH in the reaction system to 11.4 and heated to 60°C with stirring, followed by stirring for further 2 hours while maintaining the temperature at 60°C. The amounts of tungsten contained in the organic phase and in the aqueous phase were determined by inductively coupled plasma (ICP) emission spectrometry to find that tungsten was present in the organic phase and in the aqueous phase respectively in amounts of 0.051 g and 0.449 g. As a result of subsequent separation, the organic phase (31.9 g) and the aqueous phase (30.6 g) were recovered.

In a nitrogen atmosphere, the recovered aqueous phase was combined with 69.6% trioctylmethylammonium chloride (0.583 g, 1.004 mmol), toluene (21.2 g), and 85% phosphoric acid (2.85 g), and the pH in the reaction system was thereby adjusted to 2.5. The reaction system was heated to 80°C with stirring, followed by stirring for further 4 hours while maintaining the temperature at 80°C. As a result of subsequent separation, the organic phase (21.7 g) and the aqueous phase (32.3 g) were recovered.

The amounts of tungsten contained in the organic phase and in the aqueous phase were determined by inductively coupled plasma (ICP) emission spectrometry to find that tungsten was present in the organic phase and in the aqueous phase respectively in amounts of 0.449 g and 0.000 g (no tungsten remained in the aqueous phase). Thus, 90% of the initially charged amount of metallic tungsten could be recovered in the organic phase.

### Example 3

### Oxidation Reaction: Synthesis of 1,2-epoxy-4-vinylcyclohexane

In a nitrogen atmosphere at room temperature, a 100-mL four-neck flask was charged with 4-vinylcyclohexene (10.00 g, 92.4 mmol), 69.6% trioctylmethylammonium chloride (0.305 g, 0.525 mmol), sodium tungstate dihydrate (0.853 g, 2.585 mmol), disodium hydrogenphosphate dodecahydrate (0.183 g, 0.511 mmol), 85% phosphoric acid (0.270 g, 2.342 mmol), cyclohexane (30.0 g), and water (1.8 g), and the pH in the reaction system was thereby adjusted to 6.6. The reaction system was heated to 60°C with stirring and combined with a 35% aqueous hydrogen peroxide solution (8.12 g, 83.6 mmol) added dropwise over 20 minutes, followed by stirring for further one hour. The amounts of tungsten contained in the organic phase and in the aqueous phase were determined by inductively coupled plasma (ICP) emission spectrometry to find that tungsten was present in the organic phase and in the aqueous phase respectively in amounts of 0.152 g and 0.323 g. The target compound (1,2-epoxy-4-vinylcyclohexane) was found to be present in the organic phase in an amount of 5.26 g with a conversion of 51.4% and a selectivity of 89.2% in a yield of 45.8%.

### Catalyst Recovery

In a nitrogen atmosphere, the system after the completion of the reaction was combined with a 5% aqueous sodium hydroxide solution (4.18 g) to adjust the pH in the reaction system to 9.5. The reaction system with stirring was heated to 60°C, followed by stirring for further 2 hours while maintaining the temperature at 60°C. The amounts of tungsten contained in the organic phase and in the aqueous phase were determined by inductively coupled plasma (ICP) emission spectrometry to find that tungsten was present in the organic phase and in the aqueous phase respectively in amounts of 0.028 g and 0.447 g. As a result of subsequent separation, the organic phase (38.4 g) and the aqueous phase (13.4 g) were recovered.

In a nitrogen atmosphere, the recovered aqueous phase was combined with 69.6% trioctylmethylammonium chloride (0.581 g, 1.00 mmol), cyclohexane (13.4 g), and 85% phosphoric acid (0.86 g) and the the pH in the reaction system was thereby adjusted to 3.0. The reaction system was heated to 60°C with stirring and further stirred for 2 hours while maintaining the temperature at 60°C, resulting in oil precipitation. As a result of subsequent separation, an organic phase (11.3 g), an aqueous phase (12.5 g), and an oil phase (1.8 g) were recovered.

The amounts of metallic tungsten in the organic phase, in the aqueous phase, and in the oil phase were determined by inductively coupled plasma (ICP) emission spectrometry and found that metallic tungsten was present in the organic phase, in the aqueous phase, and in the oil phase respectively in amounts of 0.026 g, 0.000 g, and 0.421 g. Thus, a total of 94% of the initially charged amount of metallic tungsten could be recovered in the organic phase and in the oil phase.

### Oxidation Reaction by Recycled Catalyst

In a nitrogen atmosphere at room temperature, a 100-mL four-neck flask was charged with the organic phase and the oil phase (13.1 g) containing a tungstate (0.447 g in terms of pure tungsten) as recovered in the catalyst recovery, 3-vinylcyclohexene (9.42 g, 87.1 mmol), disodium hydrogenphosphate dodecahydrate (1.72 g, 4.80 mmol), 85% phosphoric acid (0.270 g, 2.342 mmol), cyclohexane (15.1 g), and water (1.7 g), and the pH in the reaction system was thereby adjusted to 5.7. The reaction system was heated to 60°C with stirring and combined with a 35% aqueous hydrogen peroxide solution (7.64 g, 78.6 mmol) added dropwise over 20 minutes, followed by stirring for further one hour.

The amounts of tungsten contained in the organic phase and in the aqueous phase were determined by inductively coupled plasma (ICP) emission spectrometry to find that tungsten was present in the organic phase and in the aqueous phase respectively in amounts of 0.238 g and 0.208 g. The target compound (1,2-epoxy-4-vinylcyclohexane) was found to be present in the organic phase in an amount of 5.83 g with a conversion of 58.7% and a selectivity of 91.9% in a yield of 53.9%.

### Example 4

### Oxidation Reaction: Synthesis of 3,4-epoxycyclohexylmethyl (3,4-epoxy)cyclohexanecarboxylate

In a nitrogen atmosphere at room temperature, a 100-mL four-neck flask was charged with CMCC (10.00 g, 45.4 mmol), 1-n-cetylpyridinium chloride monohydrate (0.173 g, 0.508 mmol), sodium tungstate dihydrate (0.834 g, 2.53 mmol), disodium hydrogenphosphate dodecahydrate (0.180 g, 0.501 mmol), 85% phosphoric acid (0.264 g, 2.29 mmol), toluene (30.0 g), and water (1.8 g), and the pH in the reaction system was thereby adjusted to 6.2. The reaction system with stirring was heated to 60°C and combined with a 35% aqueous hydrogen peroxide solution (13.06 g, 136.4 mmol) added dropwise over one hour, followed by stirring for further 21 hours.

The amounts of tungsten contained in the organic phase and in the aqueous phase were determined by inductively coupled plasma (ICP) emission spectrometry to find that tungsten was present in the organic phase and in the aqueous phase respectively in amounts of 0.030 g and 0.434 g. The target compound (3,4-epoxycyclohexylmethyl (3,4-epoxy)cyclohexanecarboxylate) was found to be present in the organic phase in an amount of 4.36 g with a conversion of 94.7% and a selectivity of 40.1% in a yield of 38.0%.

### Catalyst Recovery

In a nitrogen atmosphere, the system after the completion of the reaction was combined with a 5% aqueous sodium hydroxide solution (23.97 g) to adjust the pH in the reaction system to 12.0. The reaction system with stirring was heated to 40°C, followed by stirring for further 5 hours while maintaining the temperature at 40°C. The amounts of tungsten contained in the organic phase and in the aqueous phase were determined by inductively coupled plasma (ICP) emission spectrometry to find that tungsten was present in the organic phase and in the aqueous phase respectively in amounts of 0.020 g and 0.444 g. As a result of subsequent separation, the organic phase (39.9 g) and the aqueous phase (38.7 g) were recovered.

In a nitrogen atmosphere, the recovered aqueous phase was combined with 1-n-cetylpyridinium chloride monohydrate (0.340 g, 1.000 mmol), toluene (25.5 g), and 85% phosphoric acid (3.4 g), and the pH in the reaction system was thereby adjusted to 2.5. The resulting mixture with stirring was heated to 80°C, followed by stirring for further 4 hours while maintaining the temperature at 80°C. As a result of subsequent separation, the organic phase (26.0 g) and the aqueous phase (41.9 g) were recovered. The amounts of tungsten contained in the organic phase and in the aqueous phase were determined by inductively coupled plasma (ICP) emission spectrometry to find that tungsten was present in the organic phase and in the aqueous phase respectively in amounts of 0.422 g and 0.022 g. Thus, 91% of the initially charged amount of metallic tungsten could be recovered in the organic phase.

### Industrial Applicability

The oxoacid catalyst recovery method according to the present invention enables separation of the oxoacid catalyst from a reaction product by an easy procedure including pH control and separation operations alone. The method thereby requires neither filtration treatment nor adsorption treatment, can avoid recovery rate reduction with the treatments, and can efficiently recover the oxoacid catalyst. In addition, the method can also purify and recover the oxoacid catalyst by an easy procedure including pH control and separation operations alone. The method is thereby very economically advantageous, can lighten the environmental load, and can significantly contribute to the green chemistry. In general, a catalyst immobilized typically on a carrier suffers from deterioration in catalytic activity. However, the present invention eliminates the need of immobilizing the oxoacid catalyst typically on a carrier, can thereby prevent deterioration in catalytic activity with the immobilization of the catalyst typically on a carrier, and allows the oxoacid catalyst to maintain its catalytic activity at high level.

## Claims

1. A method for recovering an oxoacid catalyst, the oxoacid catalyst having been used in a reaction for oxidizing an organic compound with hydrogen peroxide in an aqueous/organic solvent two-phase reaction system, the method comprising
Step 1 of adjusting a pH in the reaction system to 5.0 or higher so as to transfer the oxoacid catalyst to the aqueous phase, and removing the organic phase.

2. The method for recovering an oxoacid catalyst according to claim 1, further comprising:
Step 2 of adding an organic solvent to the aqueous phase to give an aqueous/organic solvent two-phase reaction system; and
Step 3 of adjusting a pH in the reaction system to lower than 5.0 and adding a phase transfer catalyst to the reaction system so as to transfer the oxoacid catalyst to the organic phase, and removing the aqueous phase.

3. The method for recovering an oxoacid catalyst according to one of claims 1 and 2,
wherein the oxoacid catalyst comprises an oxoacid or a salt thereof, where the oxoacid comprises at least one metal atom selected from the group consisting of tungsten, manganese, molybdenum, vanadium, niobium, tantalum, chromium, and rhenium.

4. A method for producing an oxide, the method comprising:
recovering an oxoacid catalyst by the method for recovering an oxoacid catalyst according to any one of claims 1 to 3, the oxoacid catalyst having been used in a reaction for oxidizing an organic compound with hydrogen peroxide in an aqueous/organic solvent two-phase reaction system; and
oxidizing an organic compound with hydrogen peroxide in the presence of the recovered oxoacid catalyst to give the corresponding oxide.
